# EUROPÄISCHE PATENTSCHRIFT

(11) **EP 3 261 585 B1**
(45) Veröffentlichungstag und Bekanntmachung des Hinweises auf die Patenterteilung: **13.03.2019**
(21) Anmeldenummer: 16701278.0
(22) Anmeldetag: 20.01.2016
(51) Int. Cl.: A61F 2/46, A61F 2/36

(54) **AUSRICHTVORRICHTUNG ZUM AUSRICHTEN FEMORALER HÜFTSCHAFTIMPLANTATKOMPONENTEN FÜR TESTZWECKE**
ORIENTATION DEVICE FOR ORIENTING FEMORAL HIP-STEM IMPLANT COMPONENTS FOR TEST PURPOSES
DISPOSITIF D'ORIENTATION POUR L'ORIENTATION DE COMPOSANTS DE L'IMPLANT DE LA TIGE FÉMORALE À DES FINS DE TEST

(30) Priorität: 24.02.2015 DE 102015102588
(43) Veröffentlichungstag der Anmeldung: 03.01.2018
(73) Patentinhaber: Waldemar Link GmbH & Co. KG, 22339 Hamburg (DE)
(72) Erfinder: DMUSCHEWSKY, Klaus, 22397 Hamburg (DE)
(74) Vertreter: Raffay & Fleck
(86) Internationale Anmeldenummer: PCT/EP2016/051163
(87) Internationale Veröffentlichungsnummer: WO 2016/134891

(56) Entgegenhaltungen:
- WO-A1-94/08218
- DE-A1- 3 123 437
- US-A1- 2014 053 655

## Beschreibung

### Technisches Gebiet

Die vorliegende Erfindung betrifft eine Ausrichtvorrichtung zum Ausrichten femoraler Hüftschaftimplantatkomponenten für Testzwecke mit einem an einer Strebe vertikal verschiebbaren Kopfspanner zum Einspannen eines Kugelkopfes einer Hüftschaftimplantatkomponente, wobei der Kopfspanner um zwei zueinander senkrecht stehende Horizontalachsen verkippbar und in unterschiedlichen Kippstellungen bezüglich der Horizontalachsen arretierbar ist.

### Stand der Technik

Hüftschaftimplantatkomponenten müssen für deren klinische Zulassung unterschiedliche Spezifikationen erfüllen und Tests durchlaufen und bestehen. Zu diesen Tests gehört ein Belastungstest, für den die Hüftschaftimplantatkomponente mit ihrem Schaftabschnitt in bestimmter Ausrichtung in einer Aufnahme eingesetzt und dort in einem aushärtenden Haltemedium fixiert wird, im Rahmen dessen dann auf den Kugelkopf der Hüftschaftimplantatkomponente ein in Vertikalrichtung geführter Druck bzw. eine in dieser Richtung wirkende Anpresskraft ausgeübt wird. Bei diesem Belastungstest muss die Hüftschaftimplantatkomponente eine auflastende Mindestkraft schadlos überstehen können.

Für die Durchführung dieses Tests ist eine nach vorgegebenen Vorschriften vorzunehmende exakte Orientierung des Implantates im Raum und damit in der aushärtenden Einbettmasse vorzunehmen, und es ist auch die Füllhöhe der Einbettmasse bzw. die Höhe des frei überstehenden Abschnittes der Hüftschaftimplantatkomponente über die Oberfläche der Einbettmasse nach deren Aushärten bestimmt. Vorgaben hierzu sind in der Norm ISO 7206-4:2010 festgelegt.

Dabei gilt es insbesondere eine Schaftachse festzulegen, welche für die Ausrichtung der Hüftschaftimplantatkomponente für den Test in bestimmten Winkeln in der Frontalebene und in der seitlichen Ebene in Bezug auf eine Lastachse auszurichten ist. Die Schaftachse wird nach der durch die Norm gegebenen Definition bestimmt durch die Verbindungslinie zweier zentral im Schaft gelegener Punkte, wobei diese Punkte durch ihren jeweiligen Abstand von einer dem Kugelkopf abgewandten Schaftspitze definiert sind. Dieser Abstand ist dabei bezogen auf einen Bruchteil eines Abstandes zwischen der Schaftspitze und dem Mittelpunkt des Kugelkopfes. Ein erster Punkt, in der Norm mit K bezeichnet, liegt dabei in einem Abstand von 1/10 dieses Referenzmaßes von der Schaftspitze beabstandet im Mittelpunkt des Schaftes, ein zweiter Referenzpunkt, in der Norm L genannt, liegt dabei in einer Entfernung von 4/10 des oben genannten Referenzmaßes von der Schaftspitze im Mittelpunkt des Schaftes. Die Verbindungslinie K-L definiert die Schaftachse.

Für die richtige Ausrichtung der femoralen Hüftschaftimplantatkomponente für Testzwecke werden nun diese im Versuch tatsächlich ja im Inneren des Schaftes gelegenen Punkte von einer den Test durchführenden Person auf die Oberfläche des Schaftes projiziert markiert, und die durch diese Punkte K - L als Verbindungslinie derselben definierte Schaftachse wird bestimmt und entsprechend den Vorgaben der Norm ausgerichtet. Ein Belastungstest wird zum Beispiel mit einem dem Gerät, welches in der US 2014/0053655 offenbart wird, ähnlichem Messapparat durchgeführt. Solche Messapparate weisen ein Mittel auf, um das Messobjekt in einer Ebene auszurichten, womit im Falle eines Belastungstests von femoralen Hüftschaftimplantatkomponenten die markierte Schaftachse nach den Vorgaben der Norm ausgerichtet werden kann.

Diese letztlich mit Augenmaß durchgeführte manuelle Vorgehensweise führt zu nicht unerheblichen Abweichungen im Hinblick auf die Bestimmung der Schaftachse und damit auch bei der korrekten Ausrichtung der Hüftschaftimplantatkomponente für die weitere Festlegung in der Aushärtmasse für die Vorbereitung der Belastungstests. Diese Unsicherheiten führen nicht nur dazu, dass die Aussagen der dann durchgeführten Tests nicht in dem gewollten Maße vergleichbar und reproduzierbar sind. Häufig müssen entsprechende Belastungstests mehrfach wiederholt werden, wenn sich bei einer aufgrund der letztlich vom Augenmaß der den Test durchführenden Person abhängigen Bestimmung der Schaftachse und damit der richtigen Ausrichtung der Hüftschaftimplantatkomponente ein Versagen derselben im Test ergibt, hier mit Verifikationstests noch einmal genau zu überprüfen ist, ob dieses Versagen an Materialfehlern bzw. einer Fehlkonstruktion der Hüftschaftimplantatkomponente selbst liegt oder aber durch eine auf einer ungenauen Bestimmung des Schaftachsenverlaufes zurückzuführenden fehlerhaften Einstellung bzw. Ausrichtung der Hüftschaftimplantatkomponente im Belastungstest bedingt ist.

### Darstellung der Erfindung

Vor dem Hintergrund dieser Unzulänglichkeiten bei der Durchführung der zulassungsrelevanten Tests, genau genommen bei der exakten Ausrichtung der Hüftschaftimplantatkomponente vor dem und für den Belastungstest, besteht hier ein Bedarf, eine Möglichkeit für eine Fehler durch das Einbeziehen des Augenmaßes ausschließende, exaktere Ausrichtung der Hüftschaftimplantatkomponente, die es zu testen gilt, zu schaffen.

Mit dieser Aufgabe und deren Lösung befasst sich die vorliegende Erfindung.

Zur Lösung dieser Aufgabe schlägt die Erfindung eine Verbesserung einer Ausrichtvorrichtung zum Ausrichten femoraler Hüftschaftimplantatkomponenten für Testzwecke mit einem an einer Strebe vertikal verschiebbaren Kopfspanner zum Einspannen eines Kugelkopfes einer Hüftschaftimplantatkomponente, welcher Kopfspanner um zwei zueinander senkrecht stehende Horizontalachsen verkippbar und in unterschiedlichen Kippstellungen bezüglich der Horizontalachsen arretierbar ist, vor, die sich dadurch auszeichnet, dass die Ausrichtvorrichtung des Weiteren eine Achsschiene aufweist, mit einer in einer Längsrichtung der Achsschiene gerade verlaufenden Zeigekante und zwei in der Längsrichtung verschiebbar angeordneten Schaftklemmen zum Einspannen eines Schaftabschnittes der Hüftschaftimplantatkomponente und mit einem quer zu der Längsrichtung orientierten Anschlag für eine dem Kugelkopf der Hüftschaftimplantatkomponente gegenüberliegende Schaftspitze, wobei die Schaftklemmen derart ausgebildet sind, dass sie den Schaftabschnitt, dort wo sie anliegen jeweils zentrisch fixieren.

Mit der erfindungsgemäß der Ausrichtvorrichtung hinzugefügten Achsschiene ist ein Instrument gegeben, mit welchem die Schaftachse in ihrer tatsächlichen Lage bzw. ihrem Verlauf im Raum ohne Zuhilfenahme des Augenmaßes sehr genau bestimmt und durch die Zeigekante an der Achsschiene - jedenfalls in einer parallel verlaufenden Linie - aufgezeigt werden kann. Hierfür von wesentlicher Bedeutung sind einerseits der Anschlag an der Achsschiene, an dem beim Gebrauch derselben die Schaftspitze der auszurichtenden Hüftschaftimplantatkomponente zur Anlage kommt, und die beiden in der Längsrichtung der Achsschiene, also parallel zu der Zeigekante, verschiebbaren Schaftklemmen zum Einspannen des Schaftabschnittes der Hüftschaftimplantatkomponente, welche Schaftklemmen mit ihrem jeweiligen Spann- bzw. Klemmzentrum zu der Zeigekante einen gleichen und jeweils gleichbleibenden Abstand aufweisen. Durch die Längsverschiebbarkeit können die Schaftklemmen entsprechend den Vorgaben der Norm ISO 7206-4:2010 in dem dort genannten Abstand von 1/10 des Referenzmaßes (Abstand der Schaftspitze zum Kugelkopfzentrum) für den Punkt K und 4/10 dieses Referenzmaßes für den Punkt L justiert werden. An diesen Positionen wird dann der Schaftabschnitt der Hüftschaftimplantatkomponente mit den Schaftklemmen eingespannt, wobei durch den Umstand, dass die Schaftklemmen in einer Weise gebildet sind, dass sie den Schaftabschnitt in dem von ihnen ergriffenen Abschnitt jeweils zentrisch fixieren, diese Schaftklemmen auf die für die genaue Bestimmung der Position der Punkte K und L, deren Verbindungslinie die Lage der Schaftachse gemäß ISO 7206-4:2010 definiert, vorgegebenen Schaftmitte referenzieren.

Somit kann durch ein entsprechendes Festlegen der Achsschiene an dem Schaftabschnitt der Hüftschaftimplantatkomponente mit an dem Anschlag anliegender Schaftspitze die Lage der Schaftachse im Raum einfach durch die (parallel zu dieser Lage verlaufende) Zeigekante der Achsschiene wiedergegeben werden. Es müssen in diesem Zusammenhang also keine Punkte auf der Außenfläche des Schaftes aufgezeichnet werden, die - nach Augenmaß - eine Projektion des tatsächlich im Mittelpunkt des Schaftes gelegenen jeweiligen Punktes K bzw. L auf die Schaftaußenfläche darstellen und deren Verbindungslinie dann zudem noch so gewählt und eingestellt werden muss, dass sie etwa dem Verlauf der tatsächlichen Verbindung der im Inneren liegenden Punkte K und L entspricht. Vielmehr kann mit der erfindungsgemäß als Bestandteil der Ausrichtvorrichtung hinzugenommenen Achsschiene mit wenigen Handgriffen und schnell und auch von weniger geübtem bzw. geschultem Testpersonal eine exakte Bestimmung des Verlaufes der Schaftachse bzw. einer zu dieser Schaftachse exakt parallelen Linie, nämlich der Linie der Zeigekante, vorgenommen werden. Für die weiteren Einstellungen der Ausrichtung der femoralen Hüftschaftimplantatkomponente wird dann, wie aus dem Stand der Technik grundsätzlich bekannt, diese Linie in den wesentlichen Winkeln zu der Belastungslinie in der Frontalebene, der Ebene, in der Mittelpunkt des Kugelkopfes (Punkt C in der Notation nach der Norm ISO 7206-4:2010), und die Punkte K und L liegen (der Winkel α nach der Notation in der Norm ISO 7206-4:2010) und in der seitlichen Ebene, die zu der Ebene, in der die Punkte C, K, L liegen, senkrecht verläuft (Winkel β in der Notation der Norm ISO 7206-4:2010), eingestellt, und die Hüftschaftimplantatkomponente wird nach erfolgter Ausrichtung durch Absenken des Kopfspanners und Eintauchen des Schaftabschnittes in eine in einem Aufnahmekörper bereitgestellte, noch nicht ausgehärtete Aushärtmasse entsprechend für den durchzuführenden Test vorbereitet und in der korrekten räumlichen Lage fixiert.

Die für das zentrische Einspannen des Schaftabschnittes vorgesehenen und wesentlichen Schaftklemmen können dabei insbesondere jeweils zwei einander gegenüberliegende und aufeinander zu spannbare Spannbacken aufweisen. Mit einer solchen Ausgestaltung lässt sich ein zentrisches Einspannen besonders einfach und mit wenigen in der Vorrichtung zu integrierenden Komponenten realisieren. Die Spannbacken können dabei insbesondere jeweils ein Prismenspannprofil aufweisen mit einem Querschnitt, der im Wesentlichen die Form eines gleichschenkligen Keils aufweist. Auf diese Weise wird eine Zentrierung des Schaftes in zwei zueinander senkrechten Richtungen erreicht, so dass eine zentrierte Einspannung des Schaftes mit der jeweiligen Schaftklemme und damit eine Referenzierung auf einen im Zentrum des Schaftes gelegenen Punkt besonders effizient und sicher erreicht wird. Der Öffnungswinkel des Keils kann dabei mit besonderem Vorteil zwischen 100° und 140° gewählt sein, insbesondere 120° betragen.

Damit das zentrische Fixieren des Schaftabschnittes besonders einfach zu bewerkstelligen ist, insbesondere mit einem fixen Abstand zu der Zeigekante, sind die Spannbacken insbesondere mittels einer Gewindespindel bewegbar, wobei die Gewindespindel auf einer ersten Seite, auf der eine erste der beiden Spannbacken sitzt, ein Rechtsgewinde, auf einer zweiten Seite, auf der die zweite der beiden Spannbacken sitzt, ein Linksgewinde aufweist und wobei beide Gewinde eine betragsmäßig gleiche Steigung haben.

Da der Schaftabschnitt femoraler Hüftschaftimplantatkomponenten vielfach nicht einen exakt geraden Verlauf aufweist, sondern gekrümmt ist, können die Schaftklemmen mit Vorteil um eine quer zu der Längsrichtung der Achsschiene verlaufende Achse verdrehbar sein. So können beispielsweise zwei einander gegenüberliegende Spannbacken in einer Lage so orientiert werden, dass sie in Richtung eines an dem eingestellten Angriffspunkt liegenden Zentrums des Schaftes diesen zentriert fixieren.

Um die für eine normgerechte Bestimmung der Punkte K und L gemäß ISO 7206-4:2010 eine zentrierte Fixierung durch die Spannklemmen an exakt diesen Punkten bzw. in Bezug auf diese Punkte - also hinsichtlich deren Abstandes zu Schaftspitz - vornehmen zu können, kann an der Achsschiene mit Vorteil eine in der Längsrichtung verlaufende, einen Nullpunkt an dem Anschlag aufweisende Längenskala angebracht sein. So können die Schaftklemmen entsprechend unter Ablesen des jeweiligen Abstandes von dem Anschlag und damit für die Aufspannung des Schaftes von der Schaftspitze eingestellt und mithin an die zuvor in ihrem Abstand von der Schaftspitze ermittelte Lage der Punkte K und L gemäß ISO 7206-4:2010 angepasst werden.

Bei der erfindungsgemäßen Ausrichtvorrichtung kann der Kopfspanner eine Seitenwange mit einer ebenen Abschlussfläche aufweisen, wobei der Kopfspanner und die Achsschiene mit den Schaftklemmen derart dimensioniert sind, dass bei zueinander fluchtend ausgerichteter Abschlussfläche und Zeigekante ein Mittelpunkt eines in dem Kopfspanner aufgenommenen Hüftkopfes in gleichem Abstand zu der Anschlussfläche gelegen ist wie ein jeweiliges Fixierzentrum der Schaftklemme zu der Zeigekante. Auf diese Weise kann, wenn die Achsschiene an dem Schaftabschnitt der femoralen Hüftschaftimplantatkomponente entsprechend der Lage der Punkte K und L gemäß ISO 7206-4:2010 fixiert ist, durch Ablegen der Seitenwange mit ihrer Abschlussfläche auf einer ebenen Fläche und in Flucht bringen der Zeigekante mit dieser ebenen Fläche (also auch Ablegen der Zeigekante auf dieser ebenen Fläche) eine Ausrichtung des femoralen Hüftschaftimplantates mit der Frontalebene parallel zu dem Auflagetisch erfolgen, um so zunächst einmal eine räumliche Orientierung besser zu referenzieren und auch um den Kugelkopf korrekt in der Einspannung im Kopfspanner auszurichten.

Bestandteil der Ausrichtvorrichtung kann weiterhin eine Winkelskala sein, um über diese wenigstens einen Neigungswinkel der Zeigekante, und damit auch der Schaftachse, die zu der Zeigekante parallel verläuft, gegenüber einer Bezugsachse bestimmen zu können. Auf diese Weise können mit Hilfe der Winkelskala die Winkeleinstellungen der Winkel a und β gemäß Notation in der ISO 7206-4:2010 durchgeführt werden. Diese Winkelskala kann als ein verschiebbares Teil vorgesehen sein, welches je nach dem zu bestimmenden Winkel angeordnet und verwendet werden kann.

Die Ausrichtvorrichtung kann schließlich einen Ablagetisch aufweisen, an dem die Strebe angeordnet ist und zu dessen Oberfläche die Strebe senkrecht verläuft. So kann diese Ablagefläche nicht nur genutzt werden, um z.B. bei einer wie oben beschriebenen Ausgestaltung mit an dem Kopfspanner ausgebildeter Seitenwange mit ebener Anschlussfläche und entsprechender Dimensionierung von Kopfspanner und Achsschiene die wie oben beschriebene Nullausrichtung der Neigung β in der Seitenebene durchzuführen. Es kann zudem auf dem Ablagetisch eine Aufnahme mit der Aushärtmasse aufgestellt werden, in die dann die ausgerichtete Hüftschaftimplantatkomponente mit ihrer Schaftspitze voran eingebracht werden kann durch Absenken des Kopfspanners entlang der Strebe. Nach Aushärten der Aushärtmasse, wenn also die Hüftschaftimplantatkomponente darin sicher fixiert ist, kann dann der Kopfspanner vom Kugelkopf der Hüftschaftimplantatkomponente gelöst werden und die so in definierter Ausrichtung fest aufgenommene Hüftschaftimplantatkomponente dem eigentlichen Belastungstest zugeführt werden.

Bereits aufgrund der obigen Ausführungen und Erläuterungen sind die wesentlichen Vorteile der erfindungsgemäß gebildeten Ausrichtvorrichtung deutlich geworden, die auf eine manuelle Ausrichtung der Schaftachse nach Augenmaß vollständig verzichtet, hier vielmehr eine technische und exakte Lösung für diese Ausrichtung vorgibt.

### Kurze Beschreibung der Zeichnungen

Noch einmal weiter verdeutlicht wird die Erfindung anhand der nachfolgenden Beschreibung eines Ausführungsbeispiels anhand der beigefügten Figuren. Dabei zeigen:
Fig. 1 eine erfindungsgemäße Ausrichtvorrichtung mit einer daran angeordneten und zur Ausrichtung festgelegten Hüftschaftimplantatkomponente;

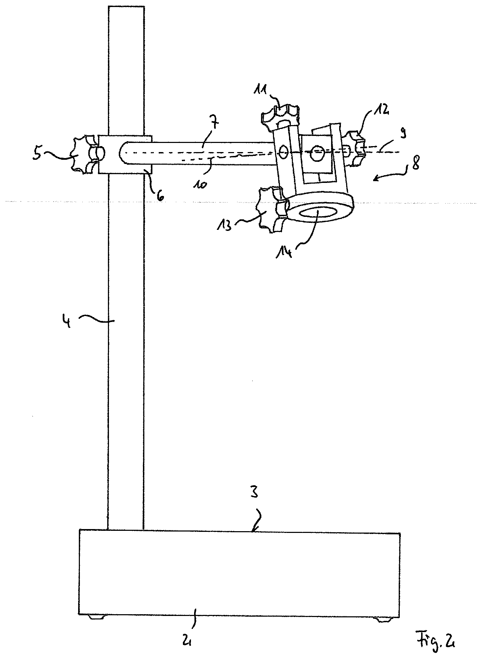

Fig. 2 einen Teil der erfindungsgemäßen Ausrichtvorrichtung, nämlich die an einem Ablagetisch angeordnete, zu dessen Oberfläche senkrecht verlaufende Strebe mit daran entlang der Längsrichtung der Strebe verlagerbar angeordnetem und um zwei Schwenkachsen verschwenkbaren und in verschiedenen Schwenkpositionen festlegbaren Kopfspanner;
Fig. 3 einen weiteren Teil der erfindungsgemäßen Ausrichtvorrichtung, nämlich die Achsschiene mit daran angeordneten, in Längsrichtung der Achsschiene verschiebbaren Schaftklemmen;
Fig. 4 eine der Schaftklemmen in vergrößerter Darstellung zur Verdeutlichung deren Eigenschaft der zentrischen Einspannung;
Fig. 5 einen Ausschnitt aus der Achsschiene mit darin verschiebbar angeordneter Schaftklemme in einer Aufsicht zur Verdeutlichung der Möglichkeit eines Verdrehens der Schaftklemme;
Fig. 6 in drei Darstellungen a bis c die Wirkweise des Einspannens verschiedener Schaftabschnitte mit unterschiedlich geformten Querschnitten jeweils in zentrisch spannender Weise mit der Schaftklemme gemäß Fig. 4;
Fig. 7 ein Beispiel einer mit ihrem Schaftabschnitt an der Achsschiene festgelegten Hüftschaftimplantatkomponente, die einen Schaftabschnitt mit stark gekrümmtem Verlauf aufweist; und
Fig. 8 eine Darstellung der erfindungsgemäßen Ausrichtvorrichtung mit auf den Ablagetisch abgesenktem und in seiner Position verdrehtem Kopfspanner zur Demonstration der Möglichkeit der so gegebenen Nullausrichtung des seitlichen Winkels β gemäß ISO 7206-4:2010.

### Weg(e) zur Ausführung der Erfindung

In den Figuren ist ein mögliches Ausführungsbeispiel einer erfindungsgemäßen Ausrichtvorrichtung schematisch dargestellt, wobei Einzelheiten der Vorrichtung in diesem Ausführungsbeispiel in einzelnen der Figuren zur weiteren Verdeutlichung vergrößert gezeigt sind. Die Figuren sind dabei keinesfalls maßstabsgerecht oder als vollständige Konstruktionszeichnungen zu verstehen. Sie stellen vielmehr Prinzipskizzen dar, die der Erläuterung der Erfindung dienen, die aber in Einzelheiten auch technische Details soweit aufzeigen und offenbaren, wie sie für die Verwirklichung der Erfindung in diesem Ausführungsbeispiel wesentlich und wichtig sind.

Anhand dieser Figuren wird nun das darin gezeigte Ausführungsbeispiel nachstehend beschrieben zum besseren Verständnis und zur weiteren Erläuterung der Erfindung.

Ein in den Figuren dargestelltes Ausführungsbeispiel einer Ausrichtvorrichtung ist allgemein mit dem Bezugszeichen 1 bezeichnet. Sie enthält einen Ablagetisch 2 mit einer im Wesentlichen horizontal verlaufenden ebenen Oberfläche 3 und einer an dem Ablagetisch 2 angeordneten, senkrecht zu der Oberfläche 3 sich erstreckenden Vertikalstrebe 4. Über eine mit einer Klemmschraube 5 in einer Vertikalposition an der Vertikalstrebe 4 festlegbaren Schiebehülse 6 ist ein an einem Auslegerarm 7 angeordneter Kopfspanner 8 mit der Vertikalstrebe 4 verbunden und kann entlang derselben in vertikaler Richtung bewegt und in der Position eingestellt werden. Der Kopfspanner 8 dient der Aufnahme eines Kugelkopfes K einer Hüftschaftimplantatkomponente H, der in dem Kopfspanner 8 nicht nur aufgenommen, sondern in diesem auch fest verspannt werden kann. Der Kopfspanner 8 kann um zwei Achsen, eine im Wesentlichen horizontal verlaufende Achse 9, die hier der Längsachse des Auslegerarms 7 entspricht, und eine ebenfalls im Wesentlichen horizontal verlaufende, zu der Achse 9 senkrechte Achse 10 verkippt werden und in einer eingestellten Verkippung fixiert werden. Zum Fixieren der jeweiligen Verkippung dienen weitere Klemmschrauben 11, 12. Mit der Klemmschraube 13 schließlich wird ein in einer Aufnahme 14 an dem Kopfspanner 8 eingesetzter Kugelkopf K verklemmt und fixiert. Dabei erfolgt die Fixierung des Kugelkopfes K in dem Aufnahmeraum 14 dergestalt, dass das Zentrum des Kugelkopfes, in der Notation der Norm ISO 7206-4:2010 C genannt, in mittiger Ausrichtung in der Aufnahme 14 zu liegen gerät.

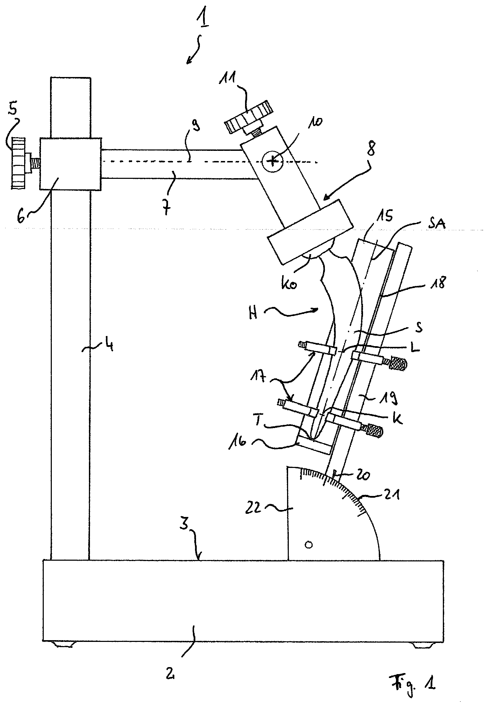

Weiterer wesentlicher Bestandteil der erfindungsgemäßen Vorrichtung 1 ist eine Achsschiene 15, die an einem Ende eine quer, insbesondere senkrecht zu einem ebenen Verlauf der Achsschiene 15 sich erstreckenden Anschlag 16 sowie zwei an der Achsschiene 15 in deren Längsrichtung verschiebbar positionierbare Schaftklemmen 17 aufweist. Diese Schaftklemmen 17 dienen in einer nachfolgend noch näher zu beschreibenden Weise einer zentrischen Erfassung und Einspannung des Schaftabschnittes S einer Hüftschaftimplantatkomponente H an zwei Punkten. Die Achsschiene 15 weist ferner eine parallel zu deren Längsrichtung angeordnete Anlagekante 18 auf. Die Anlagekante 18 der Achsschiene 15 kann, wie dies in Fig. 1 gezeigt ist, gegen eine an einem Winkelmessstück 22 drehbar angeordnete Zeigerleiste 19 eines üblichen Winkelmessers angelegt werden, um so eine Winkeleinstellung vorzunehmen. Über eine an einem Ende der Zeigerleiste 19 befindliche Zeigermarkierung 20 wird an einer an einem Winkelmessstück 22 aufgebrachten Winkelskala 21 der eingestellten Winkel z.B. gegenüber der Vertikalen anzeigt. Somit kann der Winkelmesser 19, 20, 21, 22 für das Einstellen z.B. des in der Norm ISO 7206-4:2010 mit a bezeichneten Winkels genutzt werden, wie dies die Fig. 1 zeigt.

In Fig. 1 ist auch die Lage der durch die Norm ISO 7206-4:2010 vorgegebenen Referenzpunkte K und L gezeigt, die durch ihren Abstand von der Schaftspitze T, welche an dem Anschlag 16 der Achsschiene 15 anstößt, definiert sind und mittig im Querschnitt des Schaftabschnitts liegen. Durch die zentrische Einspannung der Schaftklemmen 17 werden diese Punkte K, L referenziert. Eingezeichnet ist auch der Verlauf der über die Punkte K und L definierten Schaftachse SA, wobei deutlich wird, dass die Anlagekante 18 parallel zu dieser Schaftachse SA verläuft und mithin als Maß für die Orientierung dieser Schaftachse SA genommen werden kann.

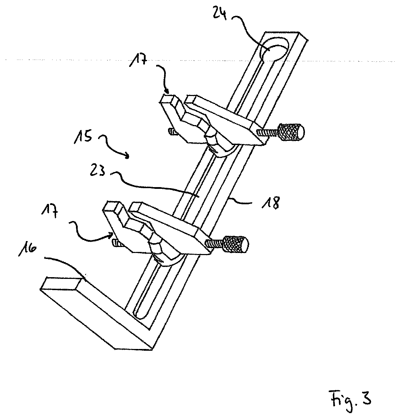

In Fig. 3 ist die Achsschiene 15 noch einmal vergrößert dargestellt. Zu erkennen ist, dass die Achsschiene 15 einen in deren Längsrichtung geführten Längsschlitz 23 aufweist, in dem die Schaftklemmen 17 längsverschieblich angeordnet sind. An einem dem Anschlag 16 gegenüberliegenden Längsende ist der Längsschlitz 23 durch eine kreisförmige Öffnung 24 erweitert. Durch diese Öffnung 24 können die Schaftklemmen 17 aus dem Längsschlitz 23 entnommen und so von der Achsschiene 15 gelöst bzw. in umgekehrter Richtung auch in den Längsschlitz 23 eingesetzt und so mit der Achsschiene 15 verbunden werden.

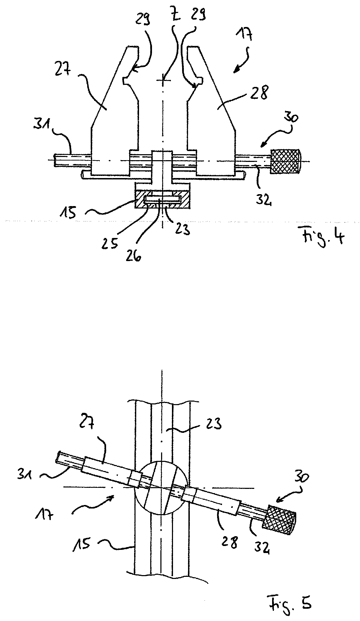

Fig. 4 zeigt in einer Querschnittsdarstellung durch die Achsschiene 15 eine vergrößerte Ansicht einer der Schaftklemmen 17. Dabei ist zu erkennen, dass der Längsschlitz 23 eine T-förmige Erweiterung 25 aufweist, in der ein tellerförmiges, im Querschnitt rundes Lagerstück 26 der Schaftklemme 17 gehalten und geführt ist. Aufgrund der im Querschnitt kreisförmigen Gestaltung des Lagerstückes 26 lässt sich die Schaftklemme 17 um eine senkrecht zu der Längsrichtung bzw. Längsachse der Achsschiene 15 verlaufende Drehachse verdrehen, wie durch Fig. 5 angedeutet ist.

Wie Fig. 4 zeigt, ist die Schaftklemme 17 als mit zwei Spannbacken 27, 28 ausgestattetes Spannelement gebildet, wobei die Spannbacken 27, 28 als Zentralspanner geformt sind und auf einen Punkt im Zentrum Z dieser Spannanordnung zu und zu diesem hin fixieren. Dafür weisen die Spannbacken 27, 28 jeweils einen keilförmigen Spannausschnitt 29 auf, dessen seitliche Schenkellinien jeweils symmetrisch verlaufen und einen Winkel von 120° einschließen. Bewegt werden die Spannbacken 27, 28 über eine Gewindespindel 30, die zwei Gewindeabschnitte, einen ersten Gewindeabschnitt 31, auf dem die erste Spannbacke 27 aufgeschraubt ist und läuft, und einen zweiten Gewindeabschnitt 32, auf dem die Spannbacke 28 aufgeschraubt ist und läuft, aufweist. Die beiden Gewindeabschnitte 31, 32 haben einen entgegengesetzten Gewindesinn mit betragsmäßig gleicher Steigung, um dadurch das zentrische Spannen der Spannbacken 27, 28 zu gewährleisten.

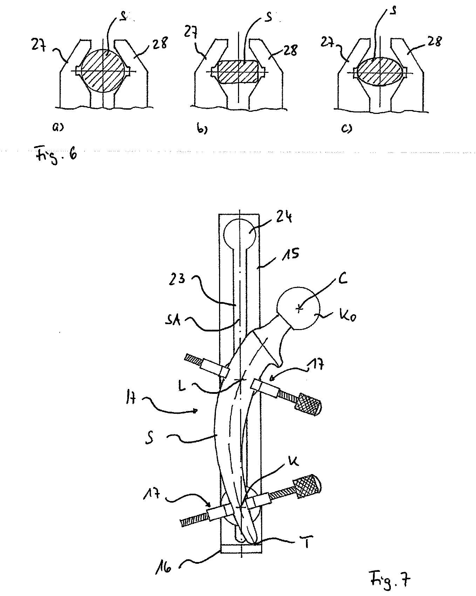

In den Figuren 6a bis 6c ist beispielhaft dargestellt, wie mit den wie in Fig. 4 gezeigt ausgebildeten Spannbacken 27, 28 und der so ausgestalteten Schaftklemme 17 Schaftabschnitte S ganz unterschiedlichen Durchmessers in zwei zueinander senkrecht dargestellten, in den Figuren jeweils eingezeichneten Raumrichtungen fixiert werden können, wobei diese Schaftabschnitte S ganz unterschiedliche Querschnittsformen aufweisen.

In Fig. 7 ist in einer Einspannsituation gezeigt, wie eine mit einem stark gekrümmten Schaftabschnitt S versehene Hüftschaftimplantatkomponente H mittels der Achsschiene 15 in Bezug auf die durch die Norm ISO 7206-4:2010 vorgegebenen Punkte K und L festgelegt und somit hinsichtlich der Orientierung und des Verlaufes der Schaftachse SA bestimmt werden kann. Zu erkennen ist hier auch wiederum, dass die Schaftspitze T an dem Anschlag 16 der Achsschiene 15 anliegt. Zu erkennen ist ferner, dass die zentrische Einspannung zur Festlegung an den zuvor bestimmten Punkten K und L dadurch erreicht werden kann, dass die Schaftklemmen 17 in der wie bereits unter Bezugnahme auf Fig. 5 erläuterten Weise relativ zu der Achsschiene 15 verdrehbar gestaltet sind. Es können also mit einer erfindungsgemäßen und wie in diesem Ausführungsbeispiel beschriebenen Achsschiene auch solche Hüftschaftimplantatkomponenten H justiert und ausgerichtet werden, die einen gekrümmten Schaftabschnitt S aufweisen.

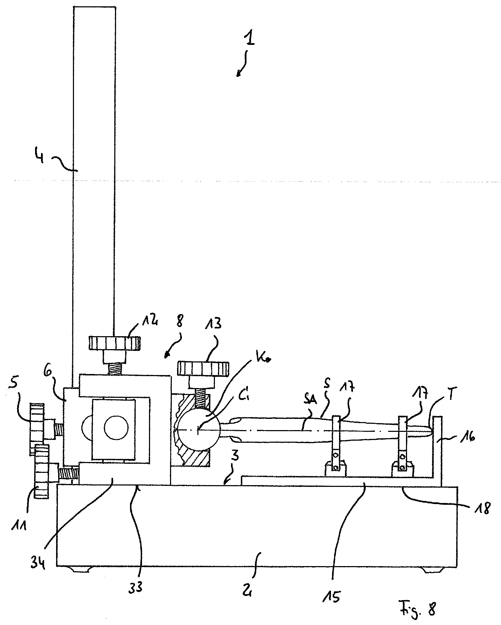

In Fig. 8 ist schließlich noch eine weitere Besonderheit der hier im Ausführungsbeispiel gezeigte Ausrichtvorrichtung dargestellt, die nämlich eine einfache
0°-Justierung des in der Norm ISO 7206-4:2010 mit β bezeichneten Winkels zwischen einer Lastachse und der Schaftachse SA in der seitlichen Ebene ermöglicht. Hierfür die ist die Kopfklemme 8 in einer Weise ausgestattet, dass eine Abschlussfläche 33 einer Seitenwange 34 eben ist, und die Achsschiene 15 ist mit ihren Schaftklemmen 17 in der Weise gestaltet, dass wenn die Abschlussfläche 33 fluchtend mit der Anlagekante 18 der Achsschiene 15 orientiert ist, was hier durch eine gemeinschaftliche plane Auflage auf der Oberfläche 3 des Ablagetisches 2 gewährleistet ist, der Abstand des Kugelkopfzentrums C von dieser Abschlussfläche 33 gleich bemessen ist wie der Abstand der Spannzentren der Schaftklemmen 17 (die in korrekt eingestellter und zugestellter Weise den Punkten K und L des Schaftabschnittes S entsprechen und somit die Schaftachse SA in ihrer Verbindungslinie definieren) zu der Anlagekante 18 gleich ist. So kann auf einfache Weise durch wie in der Fig. 8 gezeigtes Ablegen der wie dort dargestellt orientierten Vorrichtung 1 eine Nulleinstellung des Winkels β vorgenommen werden. In dieser Nulleinstellung wird dann der Kugelkopf Ko durch Festziehen der Spannschraube 13 fixiert. In der Praxis wird im Anschluss, wenn die Achsschiene 15 wie vorstehend beschrieben durch Anordnen der Schaftklemmen 17 in entsprechendem Abstand der Punkte K und L zu der Schaftspitze T, wie in die Norm ISO 7206-4:2010 vorgibt, positioniert und damit die Achsschiene 15 an dem Schaftabschnitt S der Hüftschaftimplantatkomponente angeordnet ist, durch Verschwenken des Kopfspanners 8 um die beiden Schwenkachsen 9, 10 und Einstellen der jeweiligen Schwenkwinkel (Winkel a durch Verschwenken um die Achse C, Winkel β durch Verschwenken um die Achse 9) entsprechend den normativen Vorgaben ausgerichtet. Ist diese Ausrichtung erfolgt, wird das für die Winkeleinstellung verwendete Winkelmessstück 22 vom Ablagetisch 2 entfernt und wird die Achsschiene 15 vom Schaftabschnitt S der Hüftschaftimplantatkomponente H abgenommen. Auf dem Ablagetisch 2, genauer auf dessen Oberfläche 3 wird dann ein entsprechender Implantathalter, typischerweise ein mit einer aushärtbaren Masse, z.B. einem Zement, gefülltes Gefäß abgestellt, und die Hüftschaftimplantatkomponente H wird durch Absenken des Kopfspanners 8, welches nach Lösen der Spannschraube 5 durch Herabführen der Schiebehülse 6 entlang der Vertikalstrebe 4 geschieht, in die aushärtbare Masse eingetaucht, bis die von der Norm geforderte Eintauchtiefe bzw. der normativ vorgegebene überstehende Längenbereich eingestellt sind. In dieser Position wird die aushärtbare Masse härten gelassen. Wenn dies erfolgt ist, wird der Kopfspanner 8 durch Lösen der Spannschraube 13 abgelöst und die nun in dem ausgehärteten Material in der korrekten Ausrichtung festgelegte Hüftschaftimplantatkomponente H kann dem eigentlichen Belastungstest zugeführt werden.

Um ein korrektes Einstellen der Schaftklemmen 17 hin zu den Punkten K und L zu erleichtern, kann die Achsschiene 15 mit einer entsprechenden Längenskalierung versehen sein, deren Ursprung auf Höhe des Anschlages 16 liegt und die sich entlang der Längsrichtung der Achsschiene 15 erstreckt. Auch auf der Vertikalstrebe 4 kann eine Längenskala vorgegeben sein, um somit z.B. eine Eintauchtiefe des Schaftabschnittes S der Hüftschaftimplantatkomponente H nach deren Ausrichten in ein Gefäß mit der aushärtbaren Masse besser einstellen und so genauer vornehmen zu können.

Aus der vorstehenden Beschreibung der Erfindung ist diese in ihrem erheblichen Nutzen noch einmal deutlich geworden. Die gezeigten Ausführungsbeispiele sollen die Erfindung in ihrer Tragweite und im Hinblick auf mögliche andere Umsetzungen nicht beschränken. Sie dienen lediglich der Erläuterung und dem besseren Verständnis der allgemeinen Erfindung, die in den nachstehend wiedergegebenen Ansprüchen in ihrer Allgemeinheit definiert ist.

### Bezugszeichenliste

- 1: Ausrichtvorrichtung
- 2: Ablagetisch
- 3: Oberfläche
- 4: Vertikalstrebe
- 5: Klemmschraube
- 6: Schiebehülse
- 7: Auslegerarm
- 8: Kopfspanner
- 9: Achse
- 10: Achse
- 11: Klemmschraube
- 12: Klemmschraube
- 13: Klemmschraube
- 14: Aufnahme
- 15: Achschiene
- 16: Anschlag
- 17: Schaftklemme
- 18: Anlagekante
- 19: Zeigerleiste
- 20: Zeigermarkierung
- 21: Winkelskala
- 22: Winkelmessstück
- 23: Längsschlitz
- 24: Öffnung
- 25: Erweiterung
- 26: Lagerstück
- 27: Spannbacke
- 28: Spannbacke
- 29: Spannausschnitt
- 30: Gewindespindel
- 31: Gewindeabschnitt
- 32: Gewindeabschnitt
- 33: Abschlussfläche
- 34: Seitenwange
- C: Kugelkopfzentrum
- H: Hüftschaftimplantatkomponente
- K: Referenzpunkt
- Ko: Kugelkopf
- L: Referenzpunkt
- S: Schaftabschnitt
- SA: Schaftachse
- T: Schaftspitze
- Z: Zentrum

## Patentansprüche

1. Ausrichtvorrichtung zum Ausrichten femoraler Hüftschaftimplantatkomponenten (H) für Testzwecke mit einem an einer Strebe (4) vertikal verschiebbaren Kopfspanner (8) zum Einspannen eines Kugelkopfes (Ko) einer Hüftschaftimplantatkomponente (H), wobei der Kopfspanner (8) um zwei zueinander senkrecht stehende Horizontalachsen (9, 10) verkippbar und in unterschiedlichen Kippstellungen bezüglich der Horizontalachsen (9, 10) arretierbar ist, **dadurch gekennzeichnet, dass** die Ausrichtvorrichtung (1) ferner eine Achsschiene (15) aufweist, mit einer in einer Längsrichtung der Achsschiene (15) gerade verlaufenden Zeigekante (18) und zwei in der Längsrichtung verschiebbar angeordneten Schaftklemmen (17) zum Einspannen eines Schaftabschnittes (S) der Hüftschaftimplantatkomponente (H) und mit einem quer zu der Längsrichtung orientierten Anschlag (16) für eine dem Kugelkopf (Ko) der Hüftschaftimplantatkomponente (H) gegenüberliegende Schaftsspitze (T) wobei die Schaftklemmen (17) derart ausgebildet sind, dass sie den Schaftabschnitt (S) an dem Abschnitt an dem die Schaftklemmen (17) anliegen, jeweils zentrisch fixieren.

2. Ausrichtvorrichtung nach Anspruch 1, **dadurch gekennzeichnet, dass** die Schaftklemmen (17) jeweils zwei einander gegenüberliegende und aufeinander zu spannbare Spannbacken (27, 28) aufweisen.

3. Ausrichtvorrichtung nach Anspruch 2, **dadurch gekennzeichnet, dass** die Spannbacken (27, 28) jeweils ein Prismenspannprofil (29) aufweisen mit einem Querschnitt der im Wesentlichen die Form eines gleichschenkeligen Keils aufweist.

4. Ausrichtvorrichtung nach Anspruch 3, **gekennzeichnet durch** einen Öffnungswinkel des Keils zwischen 100° und 140°, insbesondere 120°.

5. Ausrichtvorrichtung nach einem der Ansprüche 2 bis 4, **dadurch gekennzeichnet, dass** die Spannbacken (27, 28) jeweils mittels einer Gewindespindel (30) bewegbar sind, wobei jede Gewindespindel (30) auf einer ersten Seite, auf der eine erste der beiden Spannbacken (27, 28) sitzt, ein Rechtsgewinde, auf einer zweiten Seite, auf der die zweite der beiden Spannbacken (28, 27) sitzt, ein Linksgewinde aufweist, wobei beide Gewinde eine betragsmäßig gleiche Steigung aufweisen.

6. Ausrichtvorrichtung nach einem der vorhergehenden Ansprüche, **dadurch gekennzeichnet, dass** die Schaftklemmen (17) um eine quer zu der Längsrichtung verlaufende Achse verdrehbar sind.

7. Ausrichtvorrichtung nach einem der vorhergehenden Ansprüche, **dadurch gekennzeichnet, dass** an der Achsschiene (15) eine in der Längsrichtung verlaufende, einen Nullpunkt an dem Anschlag (16) aufweisende Längenskala aufweist.

8. Ausrichtvorrichtung nach einem der vorhergehenden Ansprüche, **dadurch gekennzeichnet, dass** der Kopfspanner (8) eine Seitenwange (34) mit einer ebenen Abschlussfläche (33) aufweist, wobei der Kopfspanner (8) und die Achsschiene (15) mit den Schaftklemmen (17) derart dimensioniert sind, dass bei zueinander fluchtend ausgerichteter Abschlussfläche (33) und Zeigekante (18) ein Mittelpunkt (C) eines in dem Kopfspanner (8) aufgenommenen Kugelkopfes (Ko) in gleichem Abstand zu der Abschlussfläche (33) gelegen ist, wie ein jeweiliges Fixierzentrum (Z) der Schaftklemmen (17) zu der Zeigekante (18).

9. Ausrichtvorrichtung nach einem der vorhergehenden Ansprüche, **dadurch gekennzeichnet, dass** diese weiterhin wenigstens eine Winkelskala (21) für die Einstellung eines Neigungswinkels der Zeigekante (18) gegenüber einer Bezugsachse aufweist.

10. Ausrichtvorrichtung nach einem der vorhergehenden Ansprüche, **gekennzeichnet durch** einen Ablagetisch (2), an dem die Strebe (4) angeordnet ist und zu dessen Oberfläche (3) die Strebe (4) senkrecht verläuft.

## Claims

1. Orienting device for orienting femoral hip-stem implant components (H) for test purposes, having a head clamping element (8), which is vertically displaceable on a strut (4), for clamping a ball head (Ko) of a hip-stem implant component (H) in position, wherein the head clamping element (8) is tiltable about two horizontal axes (9, 10) that are perpendicular to one another and is lockable in different tilted positions with respect to the horizontal axes (9, 10), **characterized in that** the orienting device (1) also has an axis bar (15) having an indicator edge (18) extending in a straight line in a longitudinal direction of the axis bar (15), and two stem clamps (17), arranged so as to be displaceable in the longitudinal direction, for clamping a stem portion (S) of the hip-stem implant component (H) in position, and having a stop (16), oriented transversely to the longitudinal direction, for a stem tip (T) located at the opposite end from the ball head (Ko) of the hip-stem implant component (H), wherein the stem clamps (17) are configured such that they each centrally fix the stem portion (S) at the portion against which the stem clamps (17) bear.

2. Orienting device according to Claim 1, **characterized in that** the stem clamps (17) each have two mutually opposite clamping jaws (27, 28) that are able to be tensioned towards one another.

3. Orienting device according to Claim 2, **characterized in that** the clamping jaws (27, 28) each have a prismatic clamping profile (29) having a cross section that is substantially in the form of an equilateral wedge.

4. Orienting device according to Claim 3, **characterized by** an opening angle of the wedge of between 100° and 140°, in particular 120°.

5. Orienting device according to one of Claims 2 to 4, **characterized in that** the clamping jaws (27, 28) are each movable by means of a threaded spindle (30), wherein each threaded spindle (30) has, on a first side, on which a first of the two clamping jaws (27, 28) is fitted, a right-hand thread, and on a second side, on which the second of the two clamping jaws (28, 27) is fitted, a left-hand thread, wherein the two threads have the same pitch in terms of value.

6. Orienting device according to one of the preceding claims, **characterized in that** the stem clamps (17) are rotatable about an axis extending transversely to the longitudinal direction.

7. Orienting device according to one of the preceding claims, **characterized in that** on the axis bar (15) has a length scale that extends in the longitudinal direction and has a zero point at the stop (16).

8. Orienting device according to one of the preceding claims, **characterized in that** the head clamping element (8) has a side wall (34) having a planar terminal face (33), wherein the head clamping element (8) and the axis bar (15) with the stem clamps (17) are dimensioned such that, with the terminal face (33) and indicator edge (18) aligned with one another, a centre (C) of a ball head (Ko) received in the head clamping element (8) is located at the same distance from the terminal face (33) as a respective fixing centre (Z) of the stem clamps (17) from the indicator edge (18).

9. Orienting device according to one of the preceding claims, **characterized in that** it also has at least one angle scale (21) for setting an inclination angle of the indicator edge (18) with respect to a reference axis.

10. Orienting device according to one of the preceding claims, **characterized by** a rest table (2) on which the strut (4) is arranged and to the surface (3) of which the strut (4) extends perpendicularly.

## Revendications

1. Dispositif d'orientation, destiné à orienter des composants formant implant de tige de hanche (H) fémoraux à des fins de test, comprenant un serre-tête (8) pouvant être déplacé verticalement sur un montant (4) pour serrer une tête sphérique (Ko) d'un composant formant implant de tige de hanche (H), le serre-tête (8) pouvant pivoter sur deux axes horizontaux (9, 10) perpendiculaires l'un à l'autre et pouvant être bloqué dans différentes positions de pivotement par rapport aux axes horizontaux (9, 10), **caractérisé en ce que** le dispositif d'orientation (1) comprend en outre un rail d'axe (15) qui comprend un bord de pointage (18) s'étendant de manière rectiligne dans la direction longitudinale du rail d'axe (15) et deux brides de tige (17) disposées de manière à coulisser dans la direction longitudinale et destinées à serrer la partie tige (S) du composant d'implant de tige de hanche (H) et une butée (16) orientée transversalement à la direction longitudinale et destinée à une pointe de tige (T) opposée à la tête sphérique (Ko) du composant formant implant de tige de hanche (H), les brides de tige (17) étant conçues de manière à fixer à chaque fois de manière centrée la partie de tige (S) sur laquelle les brides de tige (17) viennent en appui.

2. Dispositif d'orientation selon la revendication 1, **caractérisé en ce que** les brides de tige (17) comportent chacune deux mâchoires de serrage (27, 28) mutuellement opposées et pouvant être serrées l'une sur l'autre.

3. Dispositif d'orientation selon la revendication 2, **caractérisé en ce que** les mâchoires de serrage (27, 28) présentent chacune un profil de serrage prismatique (29) ayant une section transversale qui a sensiblement la forme d'un coin isocèle.

4. Dispositif d'orientation selon la revendication 3, **caractérisé par** un angle d'ouverture du coin compris entre 100° et 140°, en particulier à 120°.

5. Dispositif d'alignement selon l'une des revendications 2 à 4, **caractérisé en ce que** les mâchoires de serrage (27, 28) peuvent chacune être déplacées au moyen d'une broche filetée (30), chaque broche filetée (30) comportant un filetage à droite sur un premier côté sur lequel repose une première des deux mâchoires de serrage (27, 28) et un filetage à gauche sur un deuxième côté sur lequel repose la deuxième des deux mâchoires de serrage (28, 27), les deux filetages ayant un même pas en valeur absolue.

6. Dispositif d'orientation selon l'une des revendications précédentes, **caractérisé en ce que** les brides de tige (17) peuvent tourner sur un axe s'étendant transversalement à la direction longitudinale.

7. Dispositif d'orientation selon l'une des revendications précédentes, **caractérisé en ce que** sur le rail d'axe (15) comporte une échelle de longueurs s'étendant dans la direction longitudinale et comportant un point zéro au niveau de la butée (16).

8. Dispositif d'orientation selon l'une des revendications précédentes, **caractérisé en ce que** le serre-tête (8) comporte une paroi latérale (34) pourvue d'une face d'extrémité plane (33), le serre-tête (8) et le rail d'axe (15) pourvu des brides de tige (17) sont dimensionnés de telle sorte que, lorsque la face d'extrémité (33) et le bord de pointage (18) sont alignés, le centre (C) d'une tête sphérique (Ko) reçue dans le serre-tête (8) soit placé par rapport à la face d'extrémité (33) à la même distance que le centre de fixation (Z) respectif des brides de tige (17) par rapport au bord de pointage (18).

9. Dispositif d'orientation selon l'une des revendications précédentes, **caractérisé en ce qu'**il comprend en outre au moins une échelle d'angles (21) destiné à régler l'angle d'inclinaison du bord de pointage (18) par rapport à un axe de référence.

10. Dispositif d'alignement selon l'une des revendications précédentes, **caractérisé par** une table de support (2) sur laquelle le montant (4) est disposé et le montant (4) s'étend perpendiculairement à la surface (3).
